# EUROPEAN PATENT APPLICATION

(11) **EP 4 834 875 A1**
(43) Date of publication of application: **04.11.2026**
(21) Application number: 24921970.0
(22) Date of filing: 21.11.2024
(51) Int. Cl.: C07C 63/26, C07C 27/02, C07C 51/09, C07C 69/82

(54) **HYDROLYSIS REACTION SYSTEM AND CONTROL METHOD**

(30) Priority: 31.01.2024 JP 2024012585
(71) Applicant: Mitsubishi Heavy Industries, Ltd., Chiyoda-ku Tokyo 100-8332 (JP)
(72) Inventor: MATSUBARA, Wataru, Tokyo 100-8332 (JP); MIYATA, Yasuyuki, Tokyo 100-8332 (JP); KAMITO, Ryo, Tokyo 100-8332 (JP); GENTA, Minoru, Tokyo 100-8332 (JP); ITO, Motofumi, Tokyo 100-8332 (JP)
(74) Representative: Studio Torta S.p.A.
(86) International application number: PCT/JP2024/041257
(87) International publication number: WO 2025/164051

(57) **Abstract**

Terephthalic acid is appropriately obtained from DMT while suppressing energy consumption. A hydrolysis reaction system includes: a reaction vessel to which dimethyl terephthalate and water are supplied, and in which the dimethyl terephthalate is hydrolyzed by the water to produce terephthalic acid and methanol; a discharge device that discharges a mixture of methanol and water from the reaction vessel; and a control device that controls a discharge system. The control device does not cause the discharge device to discharge the mixture during a first period from a timing at which the dimethyl terephthalate and the water are supplied to the reaction vessel to a first timing; causes the discharge device to discharge the mixture during a second period from the first timing to a second timing that is later than the first timing; and does not cause the discharge device to discharge the mixture during a third period from the second timing until terephthalic acid is discharged from the reaction vessel. The first period is shorter than the third period.

## Description

### Field

The present disclosure relates to a hydrolysis reaction system and a control method.

### Background

It is known that when terephthalic acid is prepared from dimethyl terephthalate (DMT), DMT is hydrolyzed. Patent Literature 1 discloses that heated water vapor and heated DMT are introduced into a hydrolysis reactor, the reaction product is taken out from the bottom of the hydrolysis reactor, a mixture of methanol and water is taken out from the top, the mixture is condensed, and water, which is a part of the condensate, is supplied from the top of the hydrolysis reactor.

### Citation List

### Patent Literature

Patent Literature 1: JP S57-95925 A

### Summary

### Technical Problem

However, as described in Patent Literature 1, in a case where methanol and water are discharged from the hydrolysis reactor and water is refluxed to the hydrolysis reactor, it is necessary to reflux the water at a high temperature and a high pressure, which requires excessive energy. Therefore, it is required to appropriately obtain terephthalic acid from DMT while suppressing energy consumption.

An object of the present disclosure is to provide a hydrolysis reaction system and a control method capable of appropriately obtaining terephthalic acid from DMT while suppressing energy consumption.

### Solution to Problem

To solve the above-described problem and achieve the above object, a hydrolysis reaction system includes: a reaction vessel to which dimethyl terephthalate and water are supplied, and in which the dimethyl terephthalate is hydrolyzed by the water to produce terephthalic acid and methanol; a discharge device that discharges a mixture of methanol and water from the reaction vessel; and a control device that controls the discharge device. The control device does not cause the discharge device to discharge the mixture during a first period from a timing at which the dimethyl terephthalate and the water are supplied to the reaction vessel to a first timing, the control device causes the discharge device to discharge the mixture during a second period from the first timing to a second timing that is later than the first timing, the control device does not cause the discharge device to discharge the mixture during a third period from the second timing until terephthalic acid is discharged from the reaction vessel, and the first period is shorter than the third period.

To solve the above-described problem and achieve the above object, a hydrolysis reaction system includes: a supply vessel to which dimethyl terephthalate and water are supplied; a reaction vessel that is connected to the supply vessel, to which dimethyl terephthalate and water are supplied from the supply vessel, and in which the dimethyl terephthalate is hydrolyzed by the water to produce terephthalic acid and methanol; and a discharge device that discharges a mixture of methanol and water from the reaction vessel.

To solve the above-described problem and achieve the above object, a control method for controlling a hydrolysis reaction system includes: a reaction vessel to which dimethyl terephthalate and water are supplied and in which the dimethyl terephthalate is hydrolyzed by the water to produce terephthalic acid and methanol; and a discharge device that discharges a mixture of methanol and water from the reaction vessel. The control method includes the steps of: supplying the dimethyl terephthalate and water to the reaction vessel; not causing the discharge device to discharge the mixture during a first period from a timing at which the dimethyl terephthalate and the water are supplied to the reaction vessel to a first timing; causing the discharge device to discharge the mixture during a second period from the first timing to a second timing that is later than the first timing; and causing the discharge device to discharge the mixture during a third period from the second timing until terephthalic acid is discharged from the reaction vessel. The first period is shorter than the third period.

To solve the above-described problem and achieve the above object, a control method for controlling a hydrolysis reaction system includes: a supply vessel to which dimethyl terephthalate and water are supplied; a reaction vessel connected to the supply vessel, to which dimethyl terephthalate and water are supplied from the supply vessel, and in which the dimethyl terephthalate is hydrolyzed by the water to produce terephthalic acid and methanol; and a discharge device that discharges a mixture of methanol and water from the reaction vessel. The control method includes the steps of: supplying the dimethyl terephthalate and water to the supply vessel; and causing the discharge device to discharge the mixture from the reaction vessel.

### Advantageous Effects of Invention

According to the present disclosure, it is possible to appropriately obtain terephthalic acid from DMT while suppressing energy consumption.

### Brief Description of Drawings

FIG. 1 is a schematic diagram of a polyester recycling process in the present embodiment.
FIG. 2 is a schematic diagram of a separation system according to the present embodiment.
FIG. 3 is a schematic diagram illustrating a hydrolysis reaction system according to a first embodiment.
FIG. 4 is a diagram illustrating control of a discharge device according to the first embodiment.
FIG. 5 is a flowchart illustrating a processing flow of a control section.
FIG. 6 is a schematic diagram illustrating a hydrolysis reaction system according to a second embodiment.

### Description of Embodiments

Hereinafter, a preferred embodiment of the present invention will be described in detail with reference to the accompanying drawings. Note that the present invention is not limited by the embodiment, and in a case where there are multiple embodiments, the present invention includes a combination of the embodiments.

### (Recycling process)

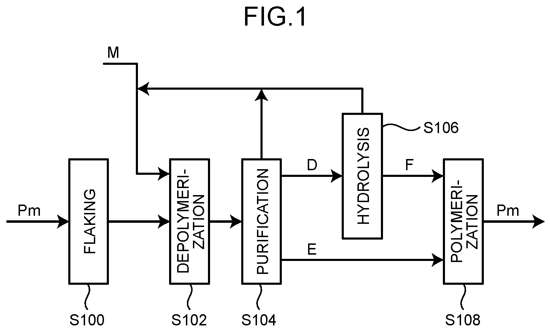

FIG. 1 is a schematic diagram of a polyester recycling process in the present embodiment. In the present embodiment, a process of recycling (regenerating) a polyester raw material Pm is performed by depolymerizing the polyester raw material Pm to form a monomer and polymerizing the monomer again. Specifically, as illustrated in FIG. 1, the polyester raw material Pm is flaked (step S100), a dissolution liquid is mixed with a reaction solvent M to depolymerize the flaked material (step S102), a monomer of the depolymerized polyester is purified (separated) to produce a monomer D derived from a carboxylic acid and a monomer E of an alcohol component (step S104), the monomer D is hydrolyzed to separate the reaction solvent M (step S106), and a monomer F produced by hydrolysis of the monomer D and the monomer E are polymerized (step S108) to regenerate the polyester raw material Pm. Note that, in the recycling process in which a separation system 1 of the present embodiment is adopted, flaking in step S100 may be omitted, or only processing of recovering the monomers D and E illustrated in step S104 and the monomer F illustrated in step S106 may be performed without performing up to processing of repolymerization as in step S108.

### (Polyester raw material)

The polyester raw material Pm to be depolymerized in the present embodiment is a substance containing polyester. The polyester raw material Pm is not particularly limited, and examples thereof include waste products such as polyethylene terephthalate (PET), polyethylene butylene terephthalate (PEBT), polybutylene terephthalate (PBT), polycyclohexane dimethyl terephthalate (PCT), polyethylene naphthalate (PEN), polybutylene naphthalate (PBN), and polycarbonate (PC). The polyester raw material Pm is not limited to those containing only a polyester component, but also contains components other than the polyester component. Examples of components other than polyester contained in the polyester raw material Pm include plastics such as polyethylene, polystyrene, polypropylene, and polyvinyl chloride other than polyester, metals, dyes, pigments, and polymerization catalysts. Clothing in which polyester and other components are woven into a fibrous form is also exemplified as the polyester raw material Pm. Hereinafter, components other than polyester contained in the polyester raw material Pm are referred to as impurities.

### (Reaction solvent)

The reaction solvent M is a solvent that reacts with polyester to depolymerize the polyester. The reaction solvent M may be, for example, at least one of methanol, ethanol, water, and ethylene glycol.

### (Monomer derived from carboxylic acid)

The monomer D derived from a carboxylic acid is a monomer having a carboxyl group, produced by a depolymerization reaction of polyester. The monomer D may be, for example, dimethyl carboxylate or diethyl carboxylate. More specifically, the monomer D is preferably a monomer of terephthalic acid, and may be, for example, dimethyl terephthalate (DMT).

### (Monomer of alcohol component)

The monomer E of an alcohol component is a monomer of an alcohol component, produced by the depolymerization reaction of polyester. The monomer E may be, for example, a dihydroxy compound (dihydric alcohol), and more specifically, may be ethylene glycol (EG).

Hereinafter, a case where the polyester is PET, the reaction solvent M is methanol, the monomer D is DMT, and the monomer E is EG will be described as an example.

### (First embodiment)

### (Separation system)

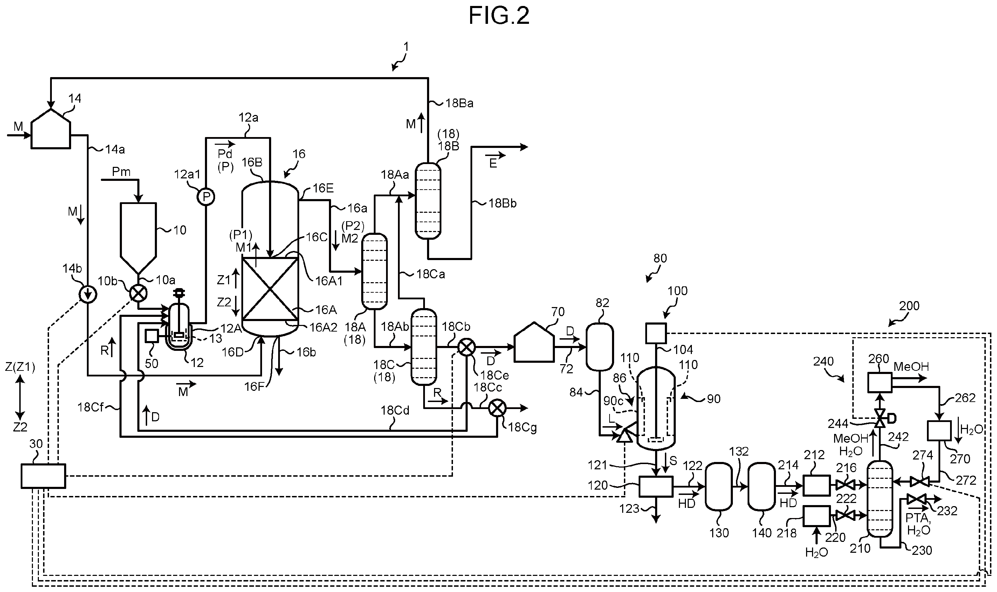

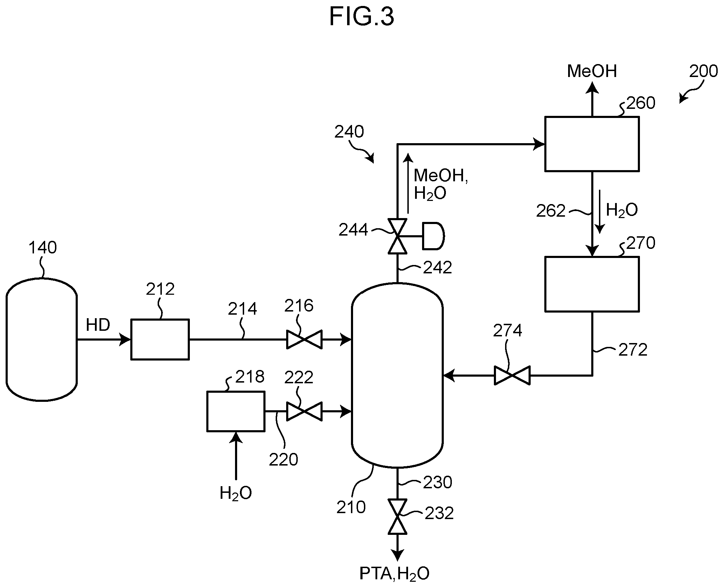

FIG. 2 is a schematic diagram of a separation system according to the first embodiment. FIG. 3 is a schematic diagram illustrating a hydrolysis reaction system according to the first embodiment. The separation system 1 according to the first embodiment is a system that monomerizes polyester contained in the polyester raw material Pm to produce monomers D and E. As illustrated in FIG. 2, the separation system 1 includes a raw material storage section 10, a dissolution section 12, a solid-liquid separation section 13, a solvent storage section 14, a reaction section 16, a separation section 18, a control section 30, a temporary storage section 70, a crystallization system 80, and a hydrolysis reaction system 200.

Hereinafter, a Z direction is a vertical direction (an up-down direction). An upward direction in the vertical direction of the Z direction is defined as a Z1 direction, and a downward direction in the vertical direction is defined as a Z2 direction.

### (Raw material storage section)

The raw material storage section 10 is a vessel into which the polyester raw material Pm is introduced and in which the polyester raw material Pm is stored. In the present embodiment, the flaked polyester raw material Pm is stored in the raw material storage section 10, but a shape and size of the polyester raw material Pm may be arbitrary. The raw material storage section 10 is connected to the dissolution section 12 via an introduction pipe 10a. The polyester raw material Pm in the raw material storage section 10 is supplied to the dissolution section 12 through the introduction pipe 10a. The introduction pipe 10a is provided with an adjustment section 10b that adjusts an amount of the polyester raw material Pm supplied from the raw material storage section 10 to the dissolution section 12. The adjustment section 10b is, for example, an on-off valve, and causes the polyester raw material Pm in the raw material storage section 10 to be supplied to the dissolution section 12 in an open state, and stops the supply of the polyester raw material Pm in the raw material storage section 10 to the dissolution section 12 in a closed state. However, the adjustment section 10b is not limited to the on-off valve, and may be any mechanism capable of adjusting the supply of the polyester raw material Pm to the dissolution section 12. Further, the polyester raw material Pm may be directly supplied to the dissolution section 12 without passing through the raw material storage section 10, the introduction pipe 10a, and the adjustment section 10b.

### (Dissolution section)

The dissolution section 12 is a vessel in which a dissolution liquid Pd is stored. The dissolution liquid Pd is a solution produced by mixing the polyester raw material Pm and the monomer D. Here, the polyester component contained in the polyester raw material Pm is dissolved in the monomer D, but impurities, which are components other than the polyester contained in the polyester raw material Pm, remain without being dissolved in the monomer D. Therefore, it can be said that the dissolution liquid Pd contains a polyester solution P, in which the polyester contained in the polyester raw material Pm is dissolved in the monomer D, and the impurities contained in the polyester raw material Pm.

The monomer D and the polyester raw material Pm are supplied to the dissolution section 12. In the dissolution section 12, while the polyester contained in the polyester raw material Pm is dissolved in the monomer D, the impurities remain without being dissolved in the monomer D, and thus a dissolution liquid Pd containing the polyester solution P and the impurities is produced. By dissolving the polyester in the monomer D in this manner, a viscosity can be reduced and fluidity can be improved, allowing the polyester to be easily discharged to the reaction section 16. Note that, the polyester solution P is not limited to a state where an entire amount of the polyester is dissolved in the monomer D, and at least a part of the polyester may remain undissolved in the monomer D. Further, in a case where there is a component that is dissolvable in the monomer D, among components other than the polyester contained in the polyester raw material Pm, the polyester solution P may also contain the component dissolved in the monomer D.

The dissolution section 12 is connected to a first reaction section 16A to be described later via an introduction pipe 12a. The dissolution liquid Pd in the dissolution section 12 passes through the introduction pipe 12a and is supplied to the first reaction section 16A. Further, the introduction pipe 12a is provided with a supply section 12a1. The supply section 12a1 is a mechanism for supplying the polyester solution P in the dissolution section 12 to the first reaction section 16A, and is a pump in the present embodiment.

In the present embodiment, the dissolution section 12 is provided with a heating section 12A. The heating section 12A heats the monomer D and the polyester raw material Pm supplied to the dissolution section 12 to a predetermined temperature by heating the inside of the dissolution section 12. The predetermined temperature is a temperature at which the polyester can be dissolved in the monomer D. By heating at a predetermined temperature in this manner, the polyester contained in the polyester raw material Pm can be appropriately dissolved in the monomer D. The predetermined temperature is preferably 140°C or higher and 300°C or lower, more preferably 160°C or higher and 280°C or lower, and still more preferably 190°C or higher and 250°C or lower. Note that, the impurities also contain a component that melts when being heated to a predetermined temperature (a temperature at which the polyester can be dissolved in the monomer D). Therefore, in a case where the impurities contain a component that melts when being heated to the predetermined temperature, the impurities are contained in the dissolution liquid Pd in a partially molten state. In the present embodiment, the heating section 12A is provided in the dissolution section 12, but a position where the heating section 12A is provided is not limited thereto and may be any position.

### (Solvent storage section)

The solvent storage section 14 is a vessel into which the reaction solvent M is introduced and in which the reaction solvent M is stored. The solvent storage section 14 is connected to the reaction section 16 via an introduction pipe 14a. The reaction solvent M in the solvent storage section 14 is supplied to the reaction section 16 through the introduction pipe 14a. More specifically, the introduction pipe 14a is provided with a heating and pressurizing section 14b that pressurizes and heats the reaction solvent M. The heating and pressurizing section 14b pressurizes and heats the reaction solvent M to bring the reaction solvent M into a supercritical state or a subcritical state (pressurized gas or pressurized liquid). The reaction solvent M in a supercritical state or a subcritical state (pressurized gas or pressurized liquid) is supplied to the reaction section 16.

### (Reaction section)

The reaction section 16 is a container into which the dissolution liquid Pd and the reaction solvent M are introduced and in which the polyester in the dissolution liquid Pd is depolymerized. The reaction section 16 includes a first reaction section 16A and a second reaction section 16B.

### (First reaction section)

The first reaction section 16A is formed in the reaction section 16. In the present embodiment, the first reaction section 16A can be said to be a portion filled with a filling material in the reaction section 16. In the first reaction section 16A, a known filling material used in a gas-liquid or liquid-liquid contact device can be used as the filling material, and for example, a filling material similar to the filling material used in a contact device for bringing heavy oil into contact with water to extract an active component can be used. Specific examples of the filling material include a pipe made of SUS or the like, a Raschig ring, a Berl saddle, a Tellerette, and a ball.

The introduction pipe 12a is connected to the first reaction section 16A. More specifically, an introduction port 16C, which is an opening of the introduction pipe 12a through which the dissolution liquid Pd from the dissolution section 12 is introduced, is connected to the first reaction section 16A. The introduction port 16C is connected to a surface 16A1 of the first reaction section 16A in the Z1 direction (upper side in the vertical direction). The introduction pipe 12a is connected to the surface 16A1 such that the introduction port 16C opens toward the Z2 direction opposite to the Z1 direction. As described above, in the present embodiment, the introduction port 16C that opens toward the Z2 direction is connected to the surface 16A1 of the first reaction section 16A, but the present invention is not limited thereto. For example, the introduction port 16C does not necessarily have to be directly connected to the first reaction section 16A, and the introduction port 16C that opens toward the Z2 direction may be connected on the side of the Z1 direction relative to the surface 16A1 of the first reaction section 16A in the reaction section 16.

The introduction pipe 14a is connected to the reaction section 16. More specifically, an introduction port 16D, which is an opening of the introduction pipe 14a through which the reaction solvent M from the solvent storage section 14 is introduced, is connected to the reaction section 16. The introduction port 16D is connected on the side of the Z2 direction relative to a surface 16A2 of the first reaction section 16A on the side of the Z2 direction. The introduction pipe 14a is connected on the side of the Z2 direction relative to the surface 16A2 such that the introduction port 16D opens toward the Z1 direction or toward the center from a side surface. As described above, in the present embodiment, the introduction port 16D, which opens toward the Z1 direction or toward the center from the side surface, is connected on the side of the Z2 direction relative to the surface 16A2 of the first reaction section 16A, but the present invention is not limited thereto. For example, the introduction port 16D may be directly connected to the first reaction section 16A or may be connected to the surface 16A2 of the first reaction section 16A.

As described above, in the present embodiment, the introduction port 16C into which the dissolution liquid Pd is introduced opens toward the Z2 direction, and the introduction port 16D into which the reaction solvent M is introduced opens toward the Z1 direction or toward the center from the side surface. Therefore, the dissolution liquid Pd and the reaction solvent M are introduced into the first reaction section 16A in directions facing each other.

The dissolution liquid Pd introduced from the introduction port 16C into the first reaction section 16A moves on the surface of the filling material of the first reaction section 16A toward the Z2 direction. On the other hand, the reaction solvent M in a supercritical state or a subcritical state (pressurized gas or pressurized liquid) introduced from the introduction port 16D moves in the first reaction section 16A toward the Z1 direction. In the first reaction section 16A, the reaction solvent M in a supercritical state or a subcritical state (pressurized gas or pressurized liquid) comes into contact with the dissolution liquid Pd. The polyester in the dissolution liquid Pd is depolymerized (reduced in molecular weight) by the reaction solvent M, and the depolymerized polyester is extracted into the reaction solvent M in a supercritical state or a subcritical state (pressurized gas or pressurized liquid). Hereinafter, the polyester depolymerized in the first reaction section 16A is referred to as a first depolymerized polyester P1, and a mixture of the first depolymerized polyester P1 and the reaction solvent M (the reaction solvent M into which the first depolymerized polyester P1 is extracted) is referred to as a first solvent M1. The first solvent M1 containing the first depolymerized polyester P1 travels through the first reaction section 16A to the side of the Z1 direction, and is discharged to the side of the Z1 direction of the first reaction section 16A.

Note that, the first depolymerized polyester P1 includes the monomers D and E produced by depolymerizing the polyester in the dissolution liquid Pd, a monomer D originally mixed in the dissolution liquid Pd, and an oligomer produced by depolymerizing the polyester. The oligomer here can be said to be an oligomer derived from a carboxylic acid or an oligomer of an alcohol component (an oligomer derived from a carboxylic acid or an oligomer of an alcohol component having a molecular weight smaller than that of polyester) that has not been monomerized but has been depolymerized from polyester. Further, the oligomer contained in a residual substance R in the polyester solution P is also depolymerized by the reaction solvent M. Therefore, the first depolymerized polyester P1 also contains the depolymerized residual substance R. The depolymerized residual substance R is, for example, an oligomer that is contained in the residual substance and has been depolymerized or monomers D and E produced by depolymerizing the oligomer contained in the residual substance, or the like.

### (Second reaction section)

The second reaction section 16B is formed in the reaction section 16, and the second reaction section 16B is formed at a portion where the first solvent M1 is discharged from the first reaction section 16A. In the present embodiment, since the first solvent M1 is discharged to the side of the Z1 direction, the second reaction section 16B can be said to be a space formed on the side of the Z1 direction of the first reaction section 16A.

In the second reaction section 16B, the first depolymerized polyester P1 contained in the first solvent M1 is further depolymerized (reduced in molecular weight) by the reaction solvent M contained in the first solvent M1. Hereinafter, the first depolymerized polyester P1 further depolymerized in the second reaction section 16B is referred to as a second depolymerized polyester P2, and a mixture of the second depolymerized polyester P2 and the reaction solvent M (the reaction solvent M in which the second depolymerized polyester P2 is dissolved) is referred to as a second solvent M2. A discharge pipe 16a is connected to the second reaction section 16B. More specifically, a discharge port 16E, which is an opening of the discharge pipe 16a through which the second solvent M2 is discharged from the second reaction section 16B, is connected to the second reaction section 16B. The second solvent M2 containing the second depolymerized polyester P2 in the second reaction section 16B is discharged from the discharge port 16E through the discharge pipe 16a to the outside of the second reaction section 16B.

Note that, the second depolymerized polyester P2 contains the monomers D and E in the first depolymerized polyester P1, monomers D and E produced by depolymerizing the oligomer in the first depolymerized polyester P1, and an oligomer produced by depolymerizing the first depolymerized polyester P1.

A discharge pipe 16b is connected to the bottom of the reaction section 16. More specifically, a discharge port 16F, which is an opening of the discharge pipe 16b through which non-extracted matter (described later) in the reaction section 16 is discharged, is connected to the bottom of the reaction section 16. From the discharge port 16F, non-extracted matter is discharged, including impurities such as metal compounds that have not been extracted into the reaction solvent M, residues of undecomposed polyester that have not been extracted into the reaction solvent M, and the like. In other words, the non-extracted matter at the bottom of the reaction section 16 is discharged from the discharge port 16F through the discharge pipe 16b to the outside of the reaction section 16. The non-extracted matter discharged from the discharge port 16F can be said to be a component of the polyester solution P that remains in the first reaction section 16A and the second reaction section 16B without being discharged to the separation section 18 as the second solvent M2 (the reaction solvent M in which the second depolymerized polyester P2 is dissolved).

Further, the reaction section 16 may be provided with a heating section that heats the inside of the reaction section 16 and a pressurizing section that maintains a pressure inside the reaction section 16 at a predetermined value or more. The temperature inside the reaction section 16 is preferably 250°C or higher and 400°C or lower, and more preferably 250°C or higher and 350°C or lower. Further, the pressure inside the reaction section 16 is preferably 1 MPa or more and 30 MPa or less, and more preferably 6 MPa or more and 25 MPa or less. The pressurizing section and the heating section may be controlled by the control section 30.

### (Separation section)

In the separation section 18, the second solvent M2 containing the second depolymerized polyester P2 is introduced, and the second solvent M2 is separated into the reaction solvent M, the monomer D derived from a carboxylic acid contained in the second depolymerized polyester P2, the monomer E of an alcohol component contained in the second depolymerized polyester P2, and the residual substance R. The separation section 18 separates the second solvent M2 into the monomer D, the monomer E, and the residual substance R by distillation. The residual substance R is a component of the second solvent M2 other than the reaction solvent M, the monomer D, and the monomer E, and contains an oligomer.

In the present embodiment, the separation section 18 includes a first separation section 18A, a second separation section 18B, and a third separation section 18C.

The first separation section 18A is a separation column connected to the discharge pipe 16a. The second solvent M2 containing the second depolymerized polyester P2 is introduced into the first separation section 18A through the discharge pipe 16a. The first separation section 18A separates the second solvent M2 into a low-boiling component and a high-boiling component having a boiling point higher than that of the low-boiling component. For example, in the first separation section 18A, the second solvent M2 may be set to a predetermined temperature, a component that has turned into a gas may be taken as a low-boiling component, and a liquid component may be taken as a high-boiling component. Discharge pipes 18Aa and 18Ab are connected to the first separation section 18A. The low-boiling component is discharged from the discharge pipe 18Aa, and the high-boiling component is discharged from the discharge pipe 18Ab.

The second separation section 18B is a separation column connected to the first separation section 18A via the discharge pipe 18Aa. The low-boiling component is introduced into the second separation section 18B through the discharge pipe 18Aa. The second separation section 18B separates the low-boiling component into the reaction solvent M and the monomer E. Discharge pipes 18Ba and 18Bb are connected to the second separation section 18B. The reaction solvent M is discharged from the discharge pipe 18Ba, and the monomer E is discharged from the discharge pipe 18Bb. Note that the discharge pipe 18Ba is connected to the second separation section 18B and the solvent storage section 14. Therefore, the reaction solvent M discharged from the second separation section 18B is returned to the solvent storage section 14 and reused for depolymerization of polyester.

The third separation section 18C is a separation column connected to the first separation section 18A via the discharge pipe 18Ab. The high-boiling component is introduced into the third separation section 18C through the discharge pipe 18Ab. The third separation section 18C separates the high-boiling component into a further higher-boiling residual substance, a low-boiling component containing the reaction solvent M and the monomer E, and the monomer D. Discharge pipes 18Ca, 18Cb, and 18Cc are connected to the third separation section 18C. The discharge pipe 18Ca is connected to the second separation section 18B. The low-boiling component separated in the third separation section 18C is discharged to the second separation section 18B through the discharge pipe 18Ca. Further, the monomer D separated in the third separation section 18C is discharged from the discharge pipe 18Cb and introduced into the temporary storage section 70. The residual substance separated in the third separation section 18C is discharged from the discharge pipe 18Cc.

An introduction pipe 18Cd is connected to the third separation section 18C. The introduction pipe 18Cd is also connected to the dissolution section 12, and introduces the monomer D discharged from the third separation section 18C into the dissolution section 12. In the example of FIG. 2, the introduction pipe 18Cd branches off from the discharge pipe 18Cb. The introduction pipe 18Cd is provided with an adjustment section 18Ce that adjusts the amount of the monomer D supplied from the third separation section 18C to the dissolution section 12. The adjustment section 18Ce is, for example, an on-off valve, and causes the monomer D to be supplied to the dissolution section 12 in the open state, and stops the supply of the monomer D to the dissolution section 12 in the closed state. However, the adjustment section 18Ce is not limited to the on-off valve, and may be any mechanism capable of adjusting the supply of the monomer D to the dissolution section 12. Note that, in the present embodiment, the adjustment section 18Ce is provided at a branching portion of the introduction pipe 18Cd from the discharge pipe 18Cb, but the position where the adjustment section 18Ce is provided is not limited thereto and may be arbitrary. Further, the introduction pipe 18Cd does not necessarily have to be connected to the discharge pipe 18Cb, and may be directly connected to the third separation section 18C. Also, for example, a storage section (vessel) for storing the monomer D may be provided in the discharge pipe 18Cb, and the introduction pipe 18Cd may be connected to the storage section.

An introduction pipe 18Cf is connected to the third separation section 18C. The introduction pipe 18Cf is also connected to the dissolution section 12, and introduces the residual substance R discharged from the third separation section 18C into the dissolution section 12. In the example of FIG. 2, the introduction pipe 18Cf branches off from the discharge pipe 18Cc. The introduction pipe 18Cf is provided with an adjustment section 18Cg that adjusts the amount of the residual substance R supplied from the third separation section 18C to the dissolution section 12. The adjustment section 18Cg is, for example, an on-off valve, and causes the residual substance R to be supplied to the dissolution section 12 in the open state, and stops the supply of the residual substance R to the dissolution section 12 in the closed state. However, the adjustment section 18Cg is not limited to the on-off valve, and may be any mechanism capable of adjusting the supply of the residual substance R to the dissolution section 12. Note that, in the present embodiment, the adjustment section 18Cg is provided at the branching portion of the introduction pipe 18Cf from the discharge pipe 18Cc, but the position where the adjustment section 18Cg is provided is not limited thereto and may be arbitrary. Further, the introduction pipe 18Cf does not necessarily have to be connected to the discharge pipe 18Cc, and may be directly connected to the third separation section 18C.

Note that, for example, a storage section (vessel) for storing the residual substance R may be provided in the discharge pipe 18Cc, and the introduction pipe 18Cf may be connected to the storage section. Further, the introduction pipe 18Cf may be provided with a filter that collects foreign matter in the residual substance R while allowing an oligomer in the residual substance R to pass through.

### (Temporary storage section)

The temporary storage section 70 is, for example, a tank, and temporarily stores the monomer D supplied from the third separation section 18C. The temporary storage section 70 is connected to the third separation section 18C via the discharge pipe 18Cb. The temporary storage section 70 is connected to a dissolution vessel 82 to be described later via a discharge pipe 72. The temporary storage section 70 supplies the temporarily stored monomer D to the dissolution vessel 82.

### (Crystallization system)

The crystallization system 80 is a system for crystallizing the monomer D from the solution in which the monomer D separated by the separation section 18 is dissolved. The monomer D separated by the separation section 18 may contain impurities, and the crystallization system 80 crystallizes a high-purity monomer D obtained by removing the impurities from the solution in which the monomer D is dissolved. Examples of the impurities here include an isomer of the monomer D that could not be completely separated by distillation in the third separation section 18C. For example, in a case where the monomer D is DMT, the isomer of DMT is DMI (dimethyl isophthalate) derived from IPA (isophthalic acid) which is copolymer component. DMI has a boiling point close to that of DMT, and it is difficult to separate DMI even when DMI is distilled, but DMI can be separated by crystallization. In other words, in the present embodiment, a high-purity monomer D can be extracted by crystallizing the monomer D (DMT) without crystallizing DMI. Hereinafter, the high-purity monomer D extracted by crystallization is appropriately referred to as a monomer HD.

As illustrated in FIG. 2, the crystallization system 80 includes a dissolution vessel 82, an adjustment section 86, a crystallization vessel 90, a stirring section 100, a solid-liquid separation section 120, a melting vessel 130, and a molten product temporary storage tank 140. In the present embodiment, the crystallization system 80 is connected downstream of the discharge pipe 72, and extracts the monomer HD by crystallization from the dissolution liquid in which the monomer D has been dissolved, flowing through the discharge pipe 72.

### (Dissolution vessel)

The dissolution vessel 82 is a tank and stores a dissolution liquid L in which the monomer D has been dissolved. The dissolution vessel 82 is connected to the temporary storage section 70 via the discharge pipe 72. The monomer D containing impurities, which has been separated by the third separation section 18C, is introduced into the dissolution vessel 82 from the temporary storage section 70. Further, a solvent for dissolving the monomer D is introduced into the dissolution vessel 82. As a result, in the dissolution vessel 82, the monomer D is dissolved in the solvent and stored as the dissolution liquid L. Note that any liquid may be used as the solvent, but methanol is used in the present embodiment.

The dissolution vessel 82 is connected to the adjustment section 86 and the crystallization vessel 90 via an introduction pipe 84. The dissolution liquid L stored in the dissolution vessel 82 is introduced into the crystallization vessel 90 through the introduction pipe 84 and the adjustment section 86. Note that the dissolution vessel 82 is located in the Z1 direction (vertically upward) relative to the crystallization vessel 90 to be described later. More specifically, a bottom surface of the dissolution vessel 82 on the side of the Z2 direction is located in the Z1 direction relative to an opening portion 90c formed in the crystallization vessel 90. However, a positional relationship between the dissolution vessel 82 and the crystallization vessel 90 in the Z direction is not limited thereto and may be arbitrary.

### (Introduction pipe)

The introduction pipe 84 is a pipe connecting the dissolution vessel 82 and the crystallization vessel 90. The introduction pipe 84 extends in the Z2 direction from a portion connected to the dissolution vessel 82 to a portion connected to the crystallization vessel 90 (adjustment section 86 in this example). In other words, it can be said that the introduction pipe 84 extends toward the Z2 direction rather than the Z1 direction as it approaches toward the portion connected to the crystallization vessel 90 (adjustment section 86 in this example) in a section from the portion connected to the dissolution vessel 82 to the portion connected to the crystallization vessel 90 (adjustment section 86 in this example).

The dissolution liquid L in the dissolution vessel 82 flows into the crystallization vessel 90 through the introduction pipe 84 and the adjustment section 86.

### (Adjustment section)

The adjustment section 86 is a device that reduces the pressure of the dissolution liquid L. The adjustment section 86 is connected to the introduction pipe 84 and the crystallization vessel 90. The adjustment section 86 reduces the pressure of the dissolution liquid L introduced from the dissolution vessel 82 through the introduction pipe 84, and introduces the dissolution liquid L under reduced pressure into the crystallization vessel 90.

The adjustment section 86 is a pressure reducing valve in the present embodiment. The adjustment section 86 may have any structure as long as it is a pressure reducing valve, but is an angle valve in the present embodiment. In the present embodiment, the adjustment section 86 is directly connected to the crystallization vessel 90.

The dissolution liquid L introduced into the adjustment section 86 is introduced into the crystallization vessel 90 in a state where the pressure has been reduced by the adjustment section 86. Since the temperature of the dissolution liquid L reduced in pressure decreases, the monomer HD which is an object to be separated is crystallized from the reduced-pressure dissolution liquid L.

Note that, in the present embodiment, the adjustment section 86 is directly connected to the crystallization vessel 90, but the present invention is not limited thereto, and a pipe connecting the adjustment section 86 and the crystallization vessel 90 may be provided between the adjustment section 86 and the crystallization vessel 90.

### (Crystallization vessel)

The crystallization vessel 90 is a vessel into which the dissolution liquid L reduced in pressure by the adjustment section 86 is introduced, and in which an object to be separated (here, the monomer HD) is crystallized from the dissolution liquid L. The dissolution liquid L reduced in pressure by the adjustment section 86 is introduced into the crystallization vessel 90. In this manner, the dissolution liquid L reduced in pressure is introduced into the crystallization vessel 90, and the dissolution liquid L is stored under reduced pressure. In other words, the crystallization vessel 90 can be said to be a flash-type crystallization vessel in which an internal pressure is reduced more than an external pressure.

The crystallization vessel 90 is connected to a discharge pipe 121 for discharging the slurry S. Note that, the slurry S is a slurry containing the crystallized monomer HD and the dissolution liquid L from which the monomer HD has been removed.

A baffle 110 that straightens the flow of the dissolution liquid L in the crystallization vessel 90 is provided inside the crystallization vessel 90. Although the shape, mounting position, and number of the baffle 110 are arbitrary, in the present embodiment, the baffle 110 is provided on an inner wall surface of the crystallization vessel 90, and more specifically, is a plate-like member extending in the Z direction on an inner wall surface of a side wall 90b. However, the baffle 110 is not an essential component, and does not necessarily have to be provided in the crystallization vessel 90.

### (Stirring section)

The stirring section 100 is provided in the crystallization vessel 90, and is a device that rotates to stir the inside of the crystallization vessel 90 when a drive section (for example, a motor) is driven. In the present embodiment, a stirring blade is located on the side of the Z2 direction relative to the adjustment section 86. Further, the stirring blade is located on the side of the Z1 direction relative to a bottom surface portion of the crystallization vessel 90 and is provided at a position facing the bottom surface portion (that is, a position overlapping the bottom surface portion as viewed from the Z direction). Further, the stirring section 100 is preferably provided at a central position of the crystallization vessel 90 when viewed from the Z direction. In other words, it is preferable that the central axis of the stirring section 100 and the central axis of the crystallization vessel 90 coincide with each other.

### (Crystallization of monomer HD)

The crystallization system 80 crystallizes the monomer HD from the dissolution liquid L in the crystallization vessel 90 with the above configuration. In other words, when the dissolution liquid L in the dissolution vessel 82 is supplied to the adjustment section 86 through the introduction pipe 84, the dissolution liquid L is reduced in pressure by the adjustment section 86. The dissolution liquid L reduced in pressure by the adjustment section 86 is introduced into the crystallization vessel 90 through the opening portion 90c. Since the temperature of the dissolution liquid L reduced in pressure decreases, the monomer HD is crystallized in the crystallization vessel 90 and stored as the slurry S. Further, since the inside of the crystallization vessel 90 is stirred by the stirring section 100, the crystallization of the monomer HD in the crystallization vessel 90 is promoted.

### (Solid-liquid separation section)

The solid-liquid separation section 120 is connected to the crystallization vessel 90 via the discharge pipe 121. The slurry S (the dissolution liquid L in which the monomer HD has crystallized) produced in the crystallization vessel 90 is introduced into the solid-liquid separation section 120 from the crystallization vessel 90 through the discharge pipe 121. The solid-liquid separation section 120 performs solid-liquid separation on the introduced slurry S. The solid-liquid separation section 120 separates the slurry S into an object to be separated which is a solid component, and a liquid component. In the present embodiment, the solid-liquid separation section 120 separates the slurry S into the monomer HD and the dissolution liquid L from which the monomer HD has been removed. The dissolution liquid L from which the monomer HD has been removed is discharged from a discharge pipe 123 connected to the solid-liquid separation section 120 and processed. The solid-liquid separation section 120 is, for example, a centrifugal separator, and may separate the object to be separated from the dissolution liquid L by centrifugal separation in which the inside of the solid-liquid separation section 120 is stirred around a predetermined axis and the object to be separated is moved radially outward of the rotation axis. The solid-liquid separation section 120 is not limited to centrifugal separation as long as the object to be separated can be separated from the dissolution liquid L. For example, the solid-liquid separation section 120 may separate the object to be separated from the dissolution liquid L by a filter.

### (Melting vessel)

A melting vessel 130 is connected to the solid-liquid separation section 120 via a discharge pipe 122. Further, the melting vessel 130 is connected to a molten product temporary storage tank 140 via a supply pipe 132. The melting vessel 130 melts (melts) the high-purity monomer HD obtained by solid-liquid separation from the slurry S. The melting vessel 130 melts the monomer HD into a liquid state, and supplies it to the molten product temporary storage tank 140 through the supply pipe 132.

### (Molten product temporary storage tank)

The molten product temporary storage tank 140 is connected to a hydrolysis reaction system 200 to be described later via a discharge pipe 214. The molten product temporary storage tank 140 is a tank that temporarily stores the monomer HD melted in the melting vessel 130. The molten product temporary storage tank 140 supplies the stored monomer HD to the hydrolysis reaction system 200.

### (Hydrolysis reaction system)

The hydrolysis reaction system 200 is a system that is supplied with DMT and water and hydrolyzes DMT to prepare PTA (high-purity terephthalic acid). Note that, in the present embodiment, the hydrolysis reaction system 200 is connected to the crystallization system 80, and hydrolyzes the monomer HD (DMT) prepared by the crystallization system 80 to prepare PTA, but is not limited to being connected to the crystallization system 80. In other words, the hydrolysis reaction system 200 may be a system provided in any facility that is supplied with DMT and water and hydrolyzes DMT to prepare PTA.

The hydrolysis reaction system 200 includes a reaction vessel 210, a first processing device 212, a second processing device 218, and a discharge system 240.

### (First processing device)

The first processing device 212 is a device that raises the temperature of the monomer HD to a predetermined temperature and raises the pressure of the monomer HD to a predetermined pressure. In the present embodiment, the first processing device 212 is connected to the molten product temporary storage tank 140, and the monomer HD is supplied from the molten product temporary storage tank 140. The first processing device 212 raises the temperature of the monomer HD supplied from the molten product temporary storage tank 140 to a predetermined temperature and raises the pressure of the monomer HD to a predetermined pressure. The predetermined temperature and the predetermined pressure may be arbitrary values, but are preferably values suitable for a hydrolysis reaction of the monomer HD, and may be, for example, 270°C and 6.4 MPa.

The first processing device 212 is connected to the reaction vessel 210 via the discharge pipe 214, and the monomer HD whose temperature and pressure have been raised by the first processing device 212 is supplied to the reaction vessel 210 through the discharge pipe 214. In the present embodiment, the discharge pipe 214 is provided with a valve portion 216. The valve portion 216 is, for example, an on-off valve, and controls the supply of the monomer HD from the first processing device 212 to the reaction vessel 210 by opening and closing the valve. For example, when the valve portion 216 is in the open state, the monomer HD in the first processing device 212 flows through the discharge pipe 214 and is introduced into the reaction vessel 210.

### (Second processing device)

The second processing device 218 is a device that processes water for hydrolyzing the monomer HD. The second processing device 218 is supplied with water from an arbitrary device or tank, raises the temperature of the supplied water to a predetermined temperature, and raises the pressure of the water to a predetermined pressure. The predetermined temperature and the predetermined pressure may be arbitrary values, but are preferably values suitable for a hydrolysis reaction of the monomer HD, and may be, for example, 270°C and 6.4 MPa.

The second processing device 218 is connected to the reaction vessel 210 via a supply pipe 220, and the water whose temperature and pressure have been raised by the second processing device 218 is supplied to the reaction vessel 210 through the supply pipe 220. In the present embodiment, the supply pipe 220 is provided with a valve portion 222. The valve portion 222 is, for example, an on-off valve, and controls the supply of water from the second processing device 218 to the reaction vessel 210 by opening and closing the valve. For example, when the valve portion 222 is in the open state, the water in the second processing device 218 flows through the supply pipe 220 and is introduced into the reaction vessel 210.

Note that the first processing device 212 and the second processing device 218 do not necessarily have to be provided. In a case where the first processing device 212 and the second processing device 218 are not provided, the temperature and pressure of the monomer HD and water may be raised in the reaction vessel 210.

### (Reaction vessel)

The reaction vessel 210 is a vessel to which the monomer HD and water are supplied. The monomer HD and water are supplied to the reaction vessel 210 separately (in a separated state), not in a mixed state. In the present embodiment, the reaction vessel 210 is connected to the first processing device 212 via the discharge pipe 214, and is connected to the second processing device 218 via the supply pipe 220. The reaction vessel 210 is supplied with the monomer HD whose temperature and pressure have been raised from the first processing device 212, and is supplied with the water whose temperature and pressure have been raised from the second processing device 218. However, the monomer HD and water may be supplied to the reaction vessel 210 in a mixed state. In this case, it is preferable that the temperature and pressure of the mixture of the monomer HD and water are raised to a predetermined temperature and a predetermined pressure in the reaction vessel 210.

In the reaction vessel 210, DMT, which is the monomer HD, is hydrolyzed with water to produce PTA and methanol. More specifically, in the reaction vessel 210, a first reaction represented by the following formula (1) and a second reaction represented by the following formula (2) proceed as hydrolysis.

DMT+H₂O→MMT+MeOH (1)

MMT+H₂O⇔PTA+MeOH (2)

DMT in formula (1) is dimethyl terephthalate, H₂O in formulas (1) and (2) is water, MMT in formulas (1) and (2) is monomethyl terephthalate, MeOH in formulas (1) and (2) is methanol, and PTA in formula (2) is high-purity terephthalic acid. In other words, in the reaction vessel 210, DMT is hydrolyzed to produce MMT, which is an intermediate product, and methanol, and MMT is hydrolyzed to produce PTA and methanol. The first reaction is an irreversible reaction in which MMT and methanol are produced from DMT and water. On the other hand, the second reaction is a reversible reaction (equilibrium reaction) in which both a reaction in which PTA and methanol are produced from MMT and water and a reaction in which MMT and water are produced from PTA and methanol occur.

A discharge pipe 230 is connected to the reaction vessel 210. The PTA and water produced in the reaction vessel 210 flow through the discharge pipe 230. A discharge portion 232 is provided in the discharge pipe 230. The discharge portion 232 is a device that discharges the PTA and water from the inside of the reaction vessel 210. The discharge portion 232 is, for example, an on-off valve, and controls inflow and outflow of the PTA and water produced in the reaction vessel 210 by opening and closing the valve. When the discharge portion 232 is in the open state, the PTA and water in the reaction vessel 210 are discharged from the reaction vessel 210 through the discharge pipe 230. The PTA discharged from the reaction vessel 210 may be processed in a subsequent process. For example, although not illustrated, the PTA and water may be stored in a hopper after undergoing a crystallization process, a dehydration process, and a drying process.

### (Discharge system)

The discharge system 240 is connected to the reaction vessel 210, and is a system that discharges a mixture of methanol and water from the reaction vessel 210, and supplies water to the reaction vessel 210. By discharging methanol from the reaction vessel 210 by the discharge system 240, the reaction in which MMT and water are produced from PTA and methanol (the leftward reaction in formula (2)) can be suppressed in the second reaction, making it possible to appropriately prepare PTA. Further, by supplying water to the reaction vessel 210 by the discharge system 240, inhibition of the reaction in which PTA is produced and an operation for aligning a particle size distribution required in the subsequent crystallization process can be suppressed, making it possible to appropriately prepare PTA. Note that, in the present embodiment, the processing of discharging methanol and water from the reaction vessel 210 and the processing of supplying water to the reaction vessel 210 are performed by the discharge system 240, but the present invention is not limited thereto. For example, the discharge system 240 may perform only the processing of discharging methanol and water from the reaction vessel 210 without performing the processing of supplying water to the reaction vessel 210.

In the present embodiment, the discharge system 240 includes a discharge device 244, a separation device 260, and a water processing device 270.

### (Discharge device)

The discharge device 244 is connected to the reaction vessel 210, and is a device that discharges a mixture of methanol and water from the reaction vessel 210. In the present embodiment, a discharge pipe 242 is connected to the reaction vessel 210, and the discharge device 244 is a valve provided in the discharge pipe 242. For example, when being in the open state, the discharge device 244 discharges methanol and water in the reaction vessel 210 from the reaction vessel 210 through the discharge pipe 242.

### (Separation device)

The separation device 260 is a device into which a mixture of methanol and water is introduced, and that separates water from the mixture of methanol and water. The separation device 260 is connected to the discharge device 244, and separates water from the mixture of methanol and water discharged from the reaction vessel 210 by the discharge device 244. More specifically, the separation device 260 is connected to the discharge pipe 242 at a position downstream of the position where the discharge device 244 is provided in a flow direction of the mixture of methanol and water. In the separation device 260, the mixture of methanol and water is introduced from the discharge pipe 242, and water is separated from the introduced mixture. The separation device 260 may be a device that separates water from a mixture of methanol and water by any method, but for example, it may evaporate methanol by heating to separate water.

The separation device 260 is connected to the water processing device 270 via a water discharge pipe 262, and the water separated by the separation device 260 is supplied to the water processing device 270 through the water discharge pipe 262.

### (Water processing device)

The water processing device 270 is a device into which the water separated by the separation device 260 is introduced and that processes the introduced water. The water processing device 270 is connected to the separation device 260 via the water discharge pipe 262, and the water separated by the separation device 260 is introduced from the water discharge pipe 262. The water processing device 270 raises the temperature of the introduced water to a predetermined temperature, and raises the pressure of the water to a predetermined pressure. The predetermined temperature and the predetermined pressure may be arbitrary values, but are preferably values suitable for a hydrolysis reaction of the monomer HD, and may be, for example, 270°C and 6.4 MPa.

The water processing device 270 is connected to the reaction vessel 210 via a processed water discharge pipe 272. The water whose temperature and pressure have been raised by the water processing device 270 is returned to the reaction vessel 210 through the processed water discharge pipe 272. In the present embodiment, the processed water discharge pipe 272 is provided with a supply device 274. The supply device 274 is a device that supplies water processed by the water processing device 270 to the reaction vessel 210 from the water processing device 270. The supply device 274 is, for example, an on-off valve, and controls the supply of the water from the water processing device 270 to the reaction vessel 210 by opening and closing the valve. When the valve portion of the supply device 274 is in the open state, the water in the water processing device 270 flows through the processed water discharge pipe 272 and is returned to the reaction vessel 210.

Note that, the water processing device 270 may be integrated with the second processing device 218 or may be omitted. In a case where the water processing device 270 is not provided, more water is supplied to the reaction vessel 210 than the amount of water when the water processing device 270 is provided. As a result, the hydrolysis reaction system 200 can perform hydrolysis processing without any shortage of water. This also leads to preventing precipitation of PTA in the reaction vessel 210, allowing appropriate processing in the process after hydrolysis.

### (Control section)

The control section 30 is a control device that controls the separation system 1. The control section 30 controls the adjustment section 10b to control the amount of the polyester raw material Pm supplied from the raw material storage section 10 to the dissolution section 12. Further, in a case where the solid-liquid separation section 13 includes a drive section, the control section 30 controls operation of the solid-liquid separation section 13. The control section 30 controls the supply section 12a1 to control the amount of the dissolution liquid Pd supplied from the dissolution section 12 to the reaction section 16. The control section 30 controls the heating and pressurizing section 14b to set the reaction solvent M to a supercritical state or a subcritical state (pressurized gas or pressurized liquid), and controls the amount of the reaction solvent M in the supercritical state or the subcritical state (pressurized gas or pressurized liquid) supplied to the reaction section 16. The control section 30 controls the adjustment section 18Ce to control the amount of the monomer D supplied to the dissolution section 12. The control section 30 controls the adjustment section 18Cg to control the amount of the residual substance R supplied to the dissolution section 12. The control section 30 adjusts the adjustment section 86 of the crystallization system 80 to control a flow rate of the dissolution liquid L into the crystallization vessel 90 while reducing the pressure of the dissolution liquid L. The control section 30 controls the rotation of the stirring section 100. The control section 30 controls operation of the hydrolysis reaction system 200.

In the present embodiment, the control section 30 is a computer, and includes, for example, a processor including an arithmetic circuit such as a Central Processing Unit (CPU) and a storage unit that stores various types of information such as arithmetic contents by the processor and programs. The control section 30 executes control of the separation system 1 by reading programs from the storage unit.

However, the separation system 1 is not limited to being automatically controlled by the control section 30, and for example, at least a part of the processing may be controlled by an operation of a worker.

### (Operation of separation system)

Next, the operation of the separation system 1 will be described. The control section 30 controls the adjustment sections 10b and 18Ce to introduce the polyester raw material Pm and the monomer D into the dissolution section 12, and mixes the polyester raw material Pm and the monomer D in the dissolution section 12 to produce the dissolution liquid Pd. The control section 30 controls the supply section 12a1 to introduce the dissolution liquid Pd produced in the dissolution section 12 into the first reaction section 16A.

The control section 30 controls the heating and pressurizing section 14b to supply the reaction solvent M in a supercritical state or a subcritical state (pressurized gas or pressurized liquid) to the reaction section 16. The control section 30 preferably sets the reaction solvent M to 250°C or higher and 400°C or lower, and more preferably to 250°C or higher and 350°C or lower. The control section 30 preferably sets the reaction solvent M to 1 MPa or more and 30 MPa or less, and more preferably to 6 MPa or more and 25 MPa or less.

As described above, when the dissolution liquid Pd and the reaction solvent M are supplied to the reaction section 16, the polyester contained in the dissolution liquid Pd is depolymerized in the first reaction section 16A to produce the first depolymerized polyester P1. Then, in the second reaction section 16B, the first depolymerized polyester P1 is further depolymerized to produce a second solvent M2, which is a mixture of the second depolymerized polyester P2 and the reaction solvent M. The second solvent M2 is separated into the reaction solvent M, the monomer D, the monomer E, and the residual substance in the first separation section 18A, the second separation section 18B, and the third separation section 18C.

The control section 30 supplies the dissolution liquid L to the adjustment section 86 by controlling the adjustment section 86, causes the adjustment section 86 to reduce the pressure of the dissolution liquid L, and causes the dissolution liquid L reduced in pressure to be introduced into the crystallization vessel 90. Since the dissolution liquid L introduced into the crystallization vessel 90 is reduced in pressure, the monomer HD, which is an object, is crystallized from the dissolution liquid L in the crystallization vessel 90.

Further, the control section 30 controls the hydrolysis reaction system 200 to hydrolyze the monomer HD to produce PTA. Specifically, the control section 30 controls the first processing device 212 to raise the temperature and pressure of the monomer HD, and supplies the monomer HD whose temperature and pressure have been raised to the reaction vessel 210. Further, the control section 30 controls the second processing device 218 to raise the temperature and pressure of water, and supplies the water whose temperature and pressure have been raised to the reaction vessel 210. Further, the control section 30 controls the discharge device 244 to discharge the mixture of water and methanol from the reaction vessel 210. The control section 30 controls the separation device 260 to separate water from the mixture of water and methanol. The control section 30 controls the water processing device 270 to raise the temperature and pressure of water, and supplies the water whose temperature and pressure have been raised to the reaction vessel 210. As a result, in the reaction vessel 210, the monomer HD is hydrolyzed to produce PTA. Further, the control section 30 controls the discharge portion 232 to discharge PTA from the reaction vessel 210.

### (Control of hydrolysis system)

As described above, the control section 30 causes the monomer HD (DMT) and water to be introduced into the reaction vessel 210, and controls the discharge device 244 to discharge the mixture of water and methanol from the reaction vessel 210. By discharging methanol from the reaction vessel 210 in this manner, as described above, the reaction in which MMT and water are produced from PTA and methanol (the leftward reaction in formula (2)) can be suppressed, making it possible to appropriately prepare PTA. Further, in order to replenish the water discharged from the reaction vessel 210 together with methanol, the control section 30 controls the water processing device 270 to return the water whose temperature and pressure have been raised to the reaction vessel 210. As a result, inhibition of the reaction in which PTA is produced and inhibition of an operation for aligning a particle size distribution required in the subsequent crystallization process can be suppressed, making it possible to appropriately prepare PTA. However, it is necessary to raise the temperature and pressure of the water to be returned to the reaction vessel 210 in order to promote hydrolysis, and raising the temperature and pressure requires energy. On the other hand, as a result of intensive studies, the present inventors have found that by appropriately controlling discharge of the mixture of water and methanol, it is possible to suppress the amount of water discharged while discharging methanol to such an extent that PTA can be appropriately produced. As a result, PTA can be appropriately produced, and the amount of water discharged can be suppressed, so that energy used for raising the temperature and pressure of the water to be returned can be suppressed. Hereinafter, specific control details will be described.

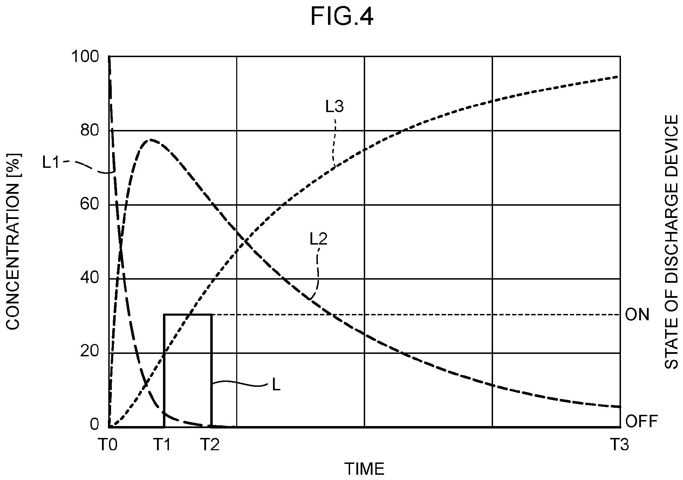

FIG. 4 is a diagram illustrating control of the discharge device according to the first embodiment. In FIG. 4, a horizontal axis represents time, a vertical axis on a right side represents the state of the discharge device 244, and a line L represents the operation of the discharge device 244. In other words, on the line L, in a case where the state of the discharge device 244 is OFF, the discharge device 244 is not discharging the mixture of water and methanol from the reaction vessel 210 (discharge is stopped). Further, on the line L, in a case where the state of the discharge device 244 is ON, the discharge device 244 is discharging the mixture of water and methanol from the reaction vessel 210. Further, a timing T0 on the horizontal axis in FIG. 4 refers to a timing at which hydrolysis of DMT starts in the reaction vessel 210. In other words, the timing T0 refers to a timing at which DMT (monomer HD) whose temperature and pressure have been raised and water whose temperature and pressure have been raised are supplied to the reaction vessel 210 (a timing at which the supply of DMT and water has been completed), and in the present embodiment, timing T0 refers to a timing at which the supply of DMT and water from the first processing device 212 and the second processing device 218 to the reaction vessel 210 has been completed. In other words, in the present embodiment, DMT and water are supplied to the reaction vessel 210 for a predetermined period, and the supply of DMT and water is stopped at the timing T0. Further, a timing T3 refers to a timing at which the discharge of PTA from the reaction vessel 210 is started, and in the present embodiment, timing T3 refers to a timing at which the PTA is discharged from the reaction vessel 210 by the discharge portion 232 after preparation of PTA is completed.

As illustrated by the line L in FIG. 4, the control section 30 stops the discharge of the mixture from the reaction vessel 210 by the discharge device 244 during a first period from the timing T0 to a first timing T1. Further, the control section 30 causes the discharge device 244 to discharge the mixture from the reaction vessel 210 during a second period from the first timing T1 to a second timing T2, which is later than the first timing T1. Further, the control section 30 stops the discharge of the mixture from the reaction vessel 210 by the discharge device 244 during a third period from the second timing T2 to the timing T3. In other words, the control section 30 causes the discharge device 244 to start discharge at the first timing T1, and causes the discharge device 244 to stop discharge at the second timing T2. Here, the first period from the timing T0 to the first timing T1 is shorter than the third period from the second timing T2 to the timing T3. In other words, the control section 30 causes methanol and water to be discharged only in an initial time period after hydrolysis has started, within an entire period from the timing T0 at which hydrolysis is started to the timing T3 at which hydrolysis is terminated. As a result, it is possible to suppress the amount of water discharged while discharging methanol to such an extent that hydrolysis can be promoted, so that PTA can be appropriately produced while suppressing energy consumption.

More specifically, as a result of intensive studies, the present inventors have grasped reaction rates in formulas (1) and (2) in the reaction vessel 210. Specifically, the present inventors have found that the reaction of formula (1) (a production reaction of MMT) rapidly proceeds at an initial stage, and the reaction of formula (2) (the equilibrium reaction for the production of MMT and PTA) proceeds relatively slowly thereafter. Therefore, as in the present embodiment, by discharging methanol and water from the first timing T1, it becomes possible to discharge methanol after MMT has been sufficiently produced, so that the production reaction of PTA can be promoted. Furthermore, by stopping the discharge of methanol and water at the relatively initial second timing T2, the amount of water discharged can be reduced and energy consumption can be suppressed. For example, the line L1 in FIG. 4 indicates an example of a concentration of DMT in the reaction vessel 210, a line L2 in FIG. 4 indicates an example of a concentration of MMT in the reaction vessel 210, and a line L3 in FIG. 4 indicates an example of a concentration of PTA in the reaction vessel 210. As illustrated in FIG. 4, by controlling the discharge device 244 as indicated by the line L, methanol can be discharged after MMT has been sufficiently produced, and the amount of water discharged can be reduced, so that it can be seen that PTA can be prepared at a high concentration while suppressing energy consumption.

Note that, the concentration of DMT in the reaction vessel 210 refers to a ratio of the mass of DMT to a total mass of DMT, MMT, and PTA. Similarly, the concentration of MMT in the reaction vessel 210 refers to the ratio of the mass of MMT to the total mass of DMT, MMT, and PTA, and the concentration of PTA in the reaction vessel 210 refers to the ratio of the mass of PTA to the total mass of DMT, MMT, and PTA.

The first timing T1 and the second timing T2 may be set to any timing at which the first period is shorter than the third period. However, in the present embodiment, the first timing T1 is preferably a timing at which the concentration of MMT in the reaction vessel 210 is 20% or more and 60% or less, more preferably a timing at which the concentration is 30% or more and 50% or less, and still more preferably a timing at which the concentration is 35% or more and 45% or less. Further, the second timing T2 is preferably a timing at which the concentration of MMT in the reaction vessel 210 becomes 0% or more and 50% or less, more preferably a timing at which the concentration becomes 0% or more and 40% or less, and still more preferably a timing at which the concentration becomes 0% or more and 30% or less. Note that, here, the concentration of MMT at each timing does not need to be actually measured, and can be calculated based on the amount, pressure, and temperature of supplied DMT and water. Further, the first timing T1 may be calculated as a timing at which the reaction to PTA reaches equilibrium based on a reaction equilibrium formula of formula (2).

Further, the ratio of a time length of the first period from the timing T0 to the first timing T1 to the period (entire period) from the timing T0 to the timing T3 is preferably 1% or more and 20% or less, more preferably 5% or more and 20% or less, and still more preferably 10% or more and 20% or less. Further, the ratio of the time length of the second period from the first timing T1 to the second timing T2 to the period (entire period) from the timing T0 to the timing T3 is preferably 1% or more and 30% or less, more preferably 5% or more and 20% or less, and still more preferably 10% or more and 20% or less. Further, each time length may be calculated from the reaction equilibrium formula of formula (2). By setting each period in this manner, PTA can be prepared at a high concentration while suppressing energy consumption.

Note that, the control section 30 may arbitrarily control the timing for returning the water whose temperature and pressure have been raised to the reaction vessel 210 from the water processing device 270. For example, the control section 30 may return the water to the reaction vessel 210 immediately after the temperature and pressure raising of the water by the water processing device 270 is completed.

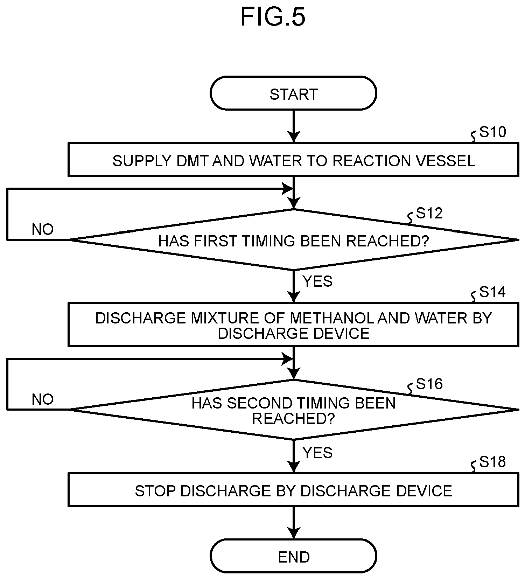

The flow of processing of the hydrolysis reaction system 200 by the control section described above will be described. FIG. 5 is a flowchart illustrating a processing flow of the control section. As illustrated in FIG. 5, the control section 30 causes DMT and water whose temperature and pressure have been raised to be supplied from the first processing device 212 and the second processing device 218 to the reaction vessel 210 (step S10). As a result, the hydrolysis reaction of DMT starts. When the first timing T1 has been reached (step S12; Yes), that is, when the first period has elapsed from the timing T0 at which the supply of DMT and water to the reaction vessel 210 is completed, the control section 30 causes the discharge device 244 to discharge the mixture of methanol and water from the reaction vessel 210 (step S14). On the other hand, when the first timing T1 has not been reached (step S12; No), the process returns to step S12, and waits for the first timing T1 to be reached while keeping the discharge by the discharge device 244 stopped.

After step S14, when the second timing T2 has been reached (step S16; Yes), the control section 30 stops the discharge of the mixture of methanol and water from the reaction vessel 210 by the discharge device 244 (step S18). Thereafter, when the timing T3 has been reached, the control section 30 controls the discharge portion 232 to discharge PTA from the reaction vessel 210, and ends the processing. On the other hand, in a case where the second timing T2 has not been reached (step S16; No), the process returns to step S16, and waits for the second timing T2 to be reached while continuing the discharge by the discharge device 244.

### (Effects of first embodiment)

As described above, in the present embodiment, the discharge of the mixture from the reaction vessel 210 by the discharge device 244 is stopped during the first period from the timing T0 to the first timing T1, the mixture is discharged from the reaction vessel 210 by the discharge device 244 during the second period from the first timing T1 to the second timing T2, and the discharge of the mixture from the reaction vessel 210 is stopped during the third period from the second timing T2 to the timing T3. As a result, it is possible to suppress the amount of water discharged while discharging methanol to such an extent that hydrolysis can be promoted, so that PTA can be appropriately produced while suppressing energy consumption.

### (Second embodiment)

Next, the second embodiment will be described. The second embodiment is different from the first embodiment in that a plurality of vessels to which DMT and water are supplied are provided in series. Components having the same functions as those in the first embodiment are denoted by the same reference numerals, and detailed descriptions thereof will be omitted.

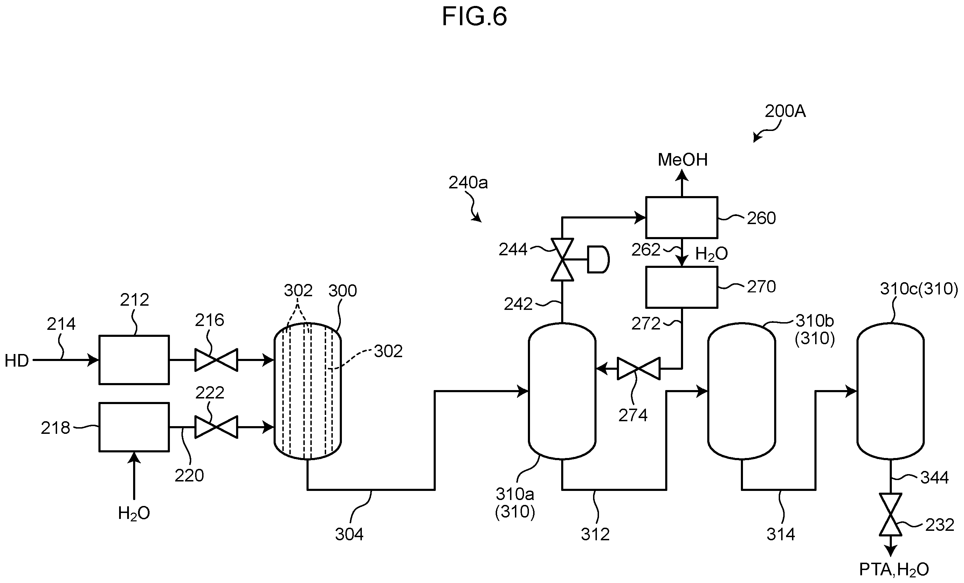

FIG. 6 is a schematic diagram illustrating a hydrolysis reaction system according to the second embodiment.

As illustrated in FIG. 6, a hydrolysis reaction system 200A according to the second embodiment includes a first processing device 212, a second processing device 218, a supply vessel 300, and a reaction vessel 310. The hydrolysis reaction system 200A according to the second embodiment may have any number of reaction vessels 310, but preferably has a plurality of reaction vessels. FIG. 6 shows an example in which three reaction vessels 310a, 310b, and 310c are provided as the reaction vessel 310.

### (Supply vessel)

The supply vessel 300 is a vessel to which DMT and water are supplied. The monomer HD and water are supplied to the supply vessel 300 separately (in a separated state), not in a mixed state. In the present embodiment, the supply vessel 300 is connected to the first processing device 212 via the discharge pipe 214, and is connected to the second processing device 218 via the supply pipe 220. The supply vessel 300 is supplied with the monomer HD whose temperature and pressure have been raised from the first processing device 212, and is supplied with the water whose temperature and pressure have been raised from the second processing device 218. However, the monomer HD and water may be supplied to the supply vessel 300 in a mixed state. In this case, it is preferable that the temperature and pressure of the mixture of the monomer HD and water are raised to a predetermined temperature and a predetermined pressure in the supply vessel 300.

The discharge system 240 is not connected to the supply vessel 300. In other words, the mixture of water and methanol is not discharged from the supply vessel 300 via the discharge device 244, and water is not supplied from the water processing device 270 to the supply vessel 300.

The supply vessel 300 preferably has a shape different from that of the reaction vessel 310 to be described later. For example, the supply vessel 300 is preferably a tubular type (plug flow type). In other words, an area of a flow path through which a fluid flows in the supply vessel 300 is preferably smaller than an area of a flow path through which a fluid flows in the reaction vessel 310. Further, a flow straightening portion 302 may be provided inside the supply vessel 300. The flow straightening portion 302 is a mechanism that straightens the flow of the fluid in the supply vessel 300, and may be, for example, a plurality of pipes arranged in parallel. As described above, since the supply vessel 300 is of a tubular type or has a flow straightening portion, it is possible to suppress the hydrolysis reaction from excessively proceeding, and it is possible to appropriately progress the hydrolysis reaction while discharging methanol by the discharge system 240 in the reaction vessel 310 in the subsequent stage.

The supply vessel 300 is connected to the reaction vessel 310 via a discharge pipe 304. In a case where a plurality of reaction vessels 310 are provided, the supply vessel 300 is connected to the reaction vessel 310 on the most upstream side in the flow direction of the fluid among the plurality of reaction vessels 310. In the example of FIG. 6, the supply vessel 300 is connected to the reaction vessel 310a. The DMT and water supplied to the supply vessel 300 are supplied to the reaction vessel 310 through the discharge pipe 304. Note that, in the supply vessel 300, a portion of the supplied DMT may be hydrolyzed. In this case, products produced by hydrolysis of the DMT (MMT, methanol, PTA, etc.) are also supplied to the reaction vessel 310. In other words, the DMT and water supplied from the supply vessel 300 to the reaction vessel 310 are not limited to only DMT and water, but also include the products produced by hydrolysis of the DMT. Further, in the supply vessel 300, when the first reaction described above is completed and all DMT has been converted to MMT, the DMT and water supplied from the supply vessel 300 to the reaction vessel 310 may refer to MMT and methanol.

### (Reaction vessel)

The reaction vessel 310 is a vessel to which DMT and water are supplied from the supply vessel 300. In the reaction vessel 310, the DMT supplied from the supply vessel 300 is hydrolyzed to produce PTA. Note that, in a case where MMT is supplied from the supply vessel 300 as a product produced by hydrolysis of the DMT, the MMT may also be hydrolyzed to produce PTA. In other words, in the reaction vessel 310, the first reaction and the second reaction described in the first embodiment are carried out. Note that, in a case where the first reaction is completed in the supply vessel 300, only the second reaction may be carried out in the reaction vessel 310.

A discharge system 240a is connected to the reaction vessel 310. Therefore, the mixture of methanol and water is discharged from the reaction vessel 310 by the discharge device 244, water is separated from the mixture by the separation device 260, and the temperature and pressure of the water separated by the separation device 260 are raised by the water processing device 270. The water whose temperature and pressure have been raised by the water processing device 270 is returned to the reaction vessel 310 through the processed water discharge pipe 272. In the present embodiment, the processed water discharge pipe 272 is provided with a supply device 274. The supply device 274 is a device that supplies water processed by the water processing device 270 to the reaction vessel 310 from the water processing device 270. The supply device 274 is, for example, an on-off valve, and controls the supply of water from the water processing device 270 to the reaction vessel 310 by opening and closing the valve. When the valve portion of the supply device 274 is in the open state, the water in the water processing device 270 flows through the processed water discharge pipe 272 and is returned to the reaction vessel 310.

Further, a discharge pipe 344 is connected to the reaction vessel 310. The PTA and water produced in the reaction vessel 310 flow through the discharge pipe 344. A discharge portion 232 is provided in the discharge pipe 344. The discharge portion 232 is a device that discharges the PTA and water from the reaction vessel 310.

Note that, in a case where a plurality of reaction vessels 310 are provided, the respective reaction vessels 310 are connected in series in the flow direction of the fluid. Then, among these reaction vessels 310, the reaction vessel 310 on the most upstream side in the flow direction of the fluid is connected to the supply vessel 300, and the reaction vessel 310 on the most downstream side in the flow direction of the fluid is connected to the discharge pipe 344. Further, the discharge system 240a may be connected to all of the respective reaction vessels 310, or the discharge system 240a may be connected to only some of the reaction vessels 310. In other words, in a case where a plurality of reaction vessels 310 are provided, the discharge system 240a may be connected to at least some of the reaction vessels 310. However, in this case, it is preferable that the discharge system 240a is connected to at least the reaction vessel 310 on the most upstream side (the reaction vessel 310 connected to the supply vessel 300). Further, the discharge system 240a does not necessarily have to be connected to the reaction vessel 310 on the most downstream side in the flow direction of the fluid. In the example of FIG. 6, the reaction vessel 310a is connected to the supply vessel 300, the reaction vessel 310b is connected to the reaction vessel 310a, and the reaction vessel 310c is connected to the reaction vessel 310b. The discharge system 240a is connected to the reaction vessel 310a, but the discharge system 240a is not connected to the reaction vessels 310b and 310c. However, the discharge system 240a may also be connected to at least one of the reaction vessel 310b and the reaction vessel 310c.

### (Control of hydrolysis system)

Next, control of the hydrolysis reaction system 200 in the second embodiment will be described. In the second embodiment, the control section 30 controls the first processing device 212 to raise the temperature and pressure of DMT, and supplies the DMT whose temperature and pressure have been raised to the supply vessel 300. Further, the control section 30 controls the second processing device 218 to raise the temperature and pressure of water, and supplies the water whose temperature and pressure have been raised to the supply vessel 300. The DMT supplied to the supply vessel 300 is supplied to the reaction vessel 310 while being hydrolyzed. The control section 30 controls the discharge device 244 to discharge the mixture of water and methanol from the reaction vessel 310. The control section 30 controls the separation device 260 to separate water from the mixture of water and methanol. The control section 30 controls the water processing device 270 to raise the temperature and pressure of water, and supplies the water whose temperature and pressure have been raised to the reaction vessel 310. As a result, in the reaction vessel 310, the reaction in which PTA is produced from MMT is promoted, and PTA is appropriately produced. Further, the control section 30 controls the discharge portion 232 to discharge the PTA from the reaction vessel 310.

As described above, in the second embodiment, the supply vessel 300 and the reaction vessel 310 are provided, and the discharge system 240 is connected to the reaction vessel 310. As a result, since methanol can be discharged in the reaction vessel 310 in which MMT has been sufficiently produced, the reaction in which PTA is produced can be promoted, and PTA can be appropriately produced. Furthermore, by not providing the discharge system 240 (discharge device 244) in the supply vessel 300, it is possible to suppress excessive discharge of water. Therefore, also in the second embodiment, since the amount of water discharged can be suppressed while methanol is discharged to such an extent that hydrolysis can be promoted, PTA can be appropriately produced while suppressing energy consumption.

Note that, in the second embodiment, it is not necessary to adjust the timing for operating the discharge device 244 as in the first embodiment. For example, in the second embodiment, the discharge device 244 may be constantly operated to constantly discharge water and methanol from the reaction vessel 310.

### (Effects of the present disclosure)

A hydrolysis reaction system according to a first aspect of the present disclosure includes: a reaction vessel 210 to which dimethyl terephthalate and water are supplied, and in which the dimethyl terephthalate is hydrolyzed by the water to produce terephthalic acid and methanol; a discharge device 244 that discharges a mixture of methanol and water from the reaction vessel 210; and a control device that controls the discharge device 244, in which the control device does not cause the discharge device 244 to discharge the mixture during a first period from a timing at which the dimethyl terephthalate and the water are supplied to the reaction vessel 210 to a first timing T1, but causes the discharge device 244 to discharge the mixture during a second period from the first timing T1 to a second timing T2, which is later than the first timing T1, and does not cause the discharge device 244 to discharge the mixture during a third period from the second timing T2 until terephthalic acid is discharged from the reaction vessel, and the first period is shorter than the third period.

As a result, it is possible to suppress the amount of water discharged while discharging methanol to such an extent that hydrolysis can be promoted, so that PTA can be appropriately produced while suppressing energy consumption. In other words, it is possible to appropriately obtain terephthalic acid from DMT while suppressing energy consumption.

In a hydrolysis reaction system according to a second aspect of the present disclosure, the first timing T1 is a timing at which the concentration of monomethyl terephthalate, which is an intermediate product obtained by hydrolysis in the reaction vessel 210, becomes 20% or more and 50% or less. As a result, it is possible to suppress the amount of water discharged while discharging methanol to such an extent that hydrolysis can be promoted, so that PTA can be appropriately produced while suppressing energy consumption.

A hydrolysis reaction system according to a third aspect of the present disclosure is the hydrolysis reaction system according to the first aspect or the second aspect, in which the second timing T2 is a timing at which the concentration of monomethyl terephthalate, which is an intermediate product obtained by hydrolysis in the reaction vessel 210, becomes 0% or more and 30% or less. As a result, it is possible to suppress the amount of water discharged while discharging methanol to such an extent that hydrolysis can be promoted, so that PTA can be appropriately produced while suppressing energy consumption.

A hydrolysis reaction system according to a fourth aspect of the present disclosure is the hydrolysis reaction system according to any one of the first to third aspects, further including: a separation device 260 that separates water from the mixture discharged by the discharge device 244; a water processing device that raises a temperature and a pressure of the water separated by the separation device 260; and a supply device 274 that supplies water whose temperature and pressure have been raised by the water processing device 270 to the reaction vessel 210. Therefore, the hydrolysis processing can be performed without any shortage of water. This also leads to preventing precipitation of PTA in the reaction vessel 210, allowing appropriate processing in the process after hydrolysis.

A hydrolysis reaction system according to a fifth aspect of the present disclosure includes: a supply vessel 300 to which dimethyl terephthalate and water are supplied; a reaction vessel 310 connected to the supply vessel 300, to which dimethyl terephthalate and water are supplied from the supply vessel 300, and in which the dimethyl terephthalate is hydrolyzed by the water to produce terephthalic acid and methanol; and a discharge device that discharges a mixture of methanol and water from the reaction vessel 310. As a result, it is possible to suppress the amount of water discharged while discharging methanol to such an extent that hydrolysis can be promoted, so that PTA can be appropriately produced while suppressing energy consumption.

A hydrolysis reaction system according to a sixth aspect of the present disclosure is the hydrolysis reaction system according to the fifth aspect, in which a plurality of reaction vessels 310 are provided in series, and the discharge device is connected to at least one or more of the reaction vessels 310. Therefore, it is possible to suppress the amount of water discharged while discharging methanol to such an extent that hydrolysis can be promoted, so that PTA can be appropriately produced while suppressing energy consumption.

A hydrolysis reaction system according to a seventh aspect of the present disclosure is the hydrolysis reaction system according to the fifth aspect or the sixth aspect, further including: a separation device 260 that separates water from the mixture discharged by the discharge device 244; and a water processing device 270 that raises the temperature and pressure of the water separated by the separation device 260 and supplies water whose temperature and pressure have been raised to the reaction vessel 310.

As a result, the hydrolysis processing can be performed without any shortage of water. Further, in the reaction vessel 310, the reaction in which PTA is produced from MMT is promoted, and PTA is appropriately produced.

A control method according to an eighth aspect of the present disclosure is a control method for controlling a hydrolysis reaction system including: a reaction vessel 210 to which dimethyl terephthalate and water are supplied, and in which the dimethyl terephthalate is hydrolyzed by the water to produce terephthalic acid and methanol; and a discharge device 244 that discharges a mixture of methanol and water from the reaction vessel 210, the control method including: a step of supplying the dimethyl terephthalate and water to the reaction vessel 210; a step of not causing the discharge device 244 to discharge the mixture during a first period from a timing at which the dimethyl terephthalate and the water are supplied to the reaction vessel 210 to a first timing T1; a step of causing the discharge device 244 to discharge the mixture during a second period from the first timing T1 to a second timing T2, which is later than the first timing T1; and a step of causing the discharge device 244 to discharge the mixture during a third period from the second timing T2 until terephthalic acid is discharged from the reaction vessel 210, in which the first period is shorter than the third period.

As a result, it is possible to suppress the amount of water discharged while discharging methanol to such an extent that hydrolysis can be promoted, so that PTA can be appropriately produced while suppressing energy consumption. In other words, it is possible to appropriately obtain terephthalic acid from DMT while suppressing energy consumption.

A control method according to a ninth aspect of the present disclosure is a control method for controlling a hydrolysis reaction system including: a supply vessel 300 to which dimethyl terephthalate and water are supplied; a reaction vessel 310 connected to the supply vessel 300, to which dimethyl terephthalate and water are supplied from the supply vessel 300, and in which the dimethyl terephthalate is hydrolyzed by the water to produce terephthalic acid and methanol; and a discharge device 244 that discharges a mixture of methanol and water from the reaction vessel 310, the control method including: a step of supplying dimethyl terephthalate and water to the supply vessel 300; and a step of causing the discharge device 244 to discharge the mixture from the reaction vessel 310.

As a result, it is possible to suppress the amount of water discharged while discharging methanol to such an extent that hydrolysis can be promoted, so that PTA can be appropriately produced while suppressing energy consumption.

Although the embodiment of the present invention has been described above, embodiments are not limited to the content of the embodiment. Further, the above-described components include those that can be easily conceived by those skilled in the art, those that are substantially the same, and those in a so-called equivalent range. Furthermore, the above-described components can be appropriately combined. Furthermore, various omissions, substitutions, or modifications of the components can be made without departing from the gist of the above-described embodiment.

### Reference Signs List

1 SEPARATION SYSTEM
12 DISSOLUTION SECTION
13 SOLID-LIQUID SEPARATION SECTION
14 SOLVENT STORAGE SECTION
16 REACTION SECTION
18 SEPARATION SECTION
30 CONTROL SECTION
82 DISSOLUTION VESSEL
86 ADJUSTMENT SECTION
90 CRYSTALLIZATION VESSEL
100 STIRRING SECTION
200, 200A HYDROLYSIS REACTION SYSTEM
210, 310, 310a, 310b, 310c REACTION VESSEL
212 FIRST PROCESSING DEVICE
218 SECOND PROCESSING DEVICE
240, 240a DISCHARGE SYSTEM
260 SEPARATION DEVICE
270 WATER PROCESSING DEVICE
300 SUPPLY VESSEL
D, E, HD MONOMER
M REACTION SOLVENT
P POLYESTER SOLUTION
Pd, L DISSOLUTION LIQUID
Pm POLYESTER RAW MATERIAL
R RESIDUAL SUBSTANCE

## Claims

1. A hydrolysis reaction system comprising:
a reaction vessel to which dimethyl terephthalate and water are supplied, and in which the dimethyl terephthalate is hydrolyzed by the water to produce terephthalic acid and methanol;
a discharge device that discharges a mixture of methanol and water from the reaction vessel; and
a control device that controls the discharge device, wherein
the control device does not cause the discharge device to discharge the mixture during a first period from a timing at which the dimethyl terephthalate and the water are supplied to the reaction vessel to a first timing,
the control device causes the discharge device to discharge the mixture during a second period from the first timing to a second timing that is later than the first timing,
the control device does not cause the discharge device to discharge the mixture during a third period from the second timing until terephthalic acid is discharged from the reaction vessel, and
the first period is shorter than the third period.

2. The hydrolysis reaction system according to claim 1, wherein the first timing is a timing at which a concentration of monomethyl terephthalate that is an intermediate product obtained by hydrolysis in the reaction vessel, becomes 20% or more and 50% or less.

3. The hydrolysis reaction system according to claim 1 or 2, wherein the second timing is a timing at which a concentration of monomethyl terephthalate that is an intermediate product obtained by hydrolysis in the reaction vessel becomes 0% or more and 30% or less.

4. The hydrolysis reaction system according to claim 1 or 2, further comprising:
a separation device that separates water from the mixture discharged by the discharge device;
a water processing device that raises a temperature and a pressure of the water separated by the separation device; and
a supply device that supplies water whose temperature and pressure have been raised by the water processing device to the reaction vessel.

5. A hydrolysis reaction system comprising:
a supply vessel to which dimethyl terephthalate and water are supplied;
a reaction vessel that is connected to the supply vessel, to which dimethyl terephthalate and water are supplied from the supply vessel, and in which the dimethyl terephthalate is hydrolyzed by the water to produce terephthalic acid and methanol; and
a discharge device that discharges a mixture of methanol and water from the reaction vessel.

6. The hydrolysis reaction system according to claim 5, wherein the reaction vessel includes a plurality of reaction vessels that are provided in series, and the discharge device is connected to at least one or more of the reaction vessels.

7. The hydrolysis reaction system according to claim 5 or 6, further comprising:
a separation device that separates water from the mixture discharged by the discharge device;
a water processing device that raises a temperature and a pressure of the water separated by the separation device; and
a supply device that supplies water whose temperature and pressure have been raised by the water processing device to the reaction vessel.

8. A control method for controlling a hydrolysis reaction system including: a reaction vessel to which dimethyl terephthalate and water are supplied and in which the dimethyl terephthalate is hydrolyzed by the water to produce terephthalic acid and methanol; and a discharge device that discharges a mixture of methanol and water from the reaction vessel, the control method comprising the steps of:
supplying the dimethyl terephthalate and water to the reaction vessel;
not causing the discharge device to discharge the mixture during a first period from a timing at which the dimethyl terephthalate and the water are supplied to the reaction vessel to a first timing;
causing the discharge device to discharge the mixture during a second period from the first timing to a second timing that is later than the first timing; and
causing the discharge device to discharge the mixture during a third period from the second timing until terephthalic acid is discharged from the reaction vessel, wherein
the first period is shorter than the third period.

9. A control method for controlling a hydrolysis reaction system including: a supply vessel to which dimethyl terephthalate and water are supplied; a reaction vessel connected to the supply vessel, to which dimethyl terephthalate and water are supplied from the supply vessel, and in which the dimethyl terephthalate is hydrolyzed by the water to produce terephthalic acid and methanol; and a discharge device that discharges a mixture of methanol and water from the reaction vessel, the control method comprising the steps of:
supplying the dimethyl terephthalate and water to the supply vessel; and
causing the discharge device to discharge the mixture from the reaction vessel.
